# EUROPEAN PATENT SPECIFICATION

(11) **EP 0 963 187 B1**
(45) Date of publication and mention of the grant of the patent: **21.11.2001**
(21) Application number: 98908375.3
(22) Date of filing: 25.02.1998
(51) Int. Cl.: A61F 13/15

(54) **A WEB-LIKE ELEMENT INTENDED FOR USE AS A LIQUID BARRIER IN ABSORBENT ARTICLES**
EIN GEWEBEARTIGES ELEMENT FÜR DEN GEBRAUCH ALS FLÜSSIGKEITSBARRIERE IN ABSORBIERENDEN ARTIKELN
ELEMENT DE TYPE VOILE DESTINE A ETRE UTILISE COMME BARRIERE A LIQUIDE DANS DES ARTICLES ABSORBANTS

(30) Priority: 26.02.1997 SE 9700694
(43) Date of publication of application: 15.12.1999
(73) Proprietor: SCA Hygiene Products AB, 405 03 Göteborg (SE)
(72) Inventor: RÖNNBERG, Peter, S-431 33 Mölndal (SE); SIMMONS, Eva, S-431 66 Mölndal (SE); GUSTAFSSON, Anders, S-427 35 Billdal (SE)
(74) Representative: Larsson, Karin
(86) International application number: SE9800342
(87) International publication number: WO9837844

(56) References cited:
- EP-A- 0 534 488
- WO-A-93/19711

## Description

### BACKGROUND

The present invention relates to a web-like element intended for use as a liquid barrier in absorbent articles, such as diapers, sanitary napkins, incontinence guards or like articles, and to an absorbent article that includes a liquid barrier formed by said element, and to the use of said element as a liquid barrier in an absorbent article.

An absorbent article includes, among other things, a bottom liquid-impermeable sheet, or backing sheet, an absorbent layer disposed on said backing sheet, and a top liquid-permeable surface sheet that is intended to lie proximal to the wearer in use. When the absorbent article concerned is a diaper or an incontinence guard, the article will also include flexible side flaps that extend laterally beyond the absorbent body on both sides thereof and elastic devices that extend longitudinally along the free side-edges of the side-flaps at least within that part of the absorbent body which forms the crotch part of the article in use, said flaps and elastic devices enabling the absorbent article to be fitted to the wearer. These elastic devices function as leg elastic when the article is worn and are intended to seal around the wearer's thighs and also to shape the article. Thus, when the article is donned, the elastic devices will be stretched and hold the side flaps tightly against the wearer. The flexible side-flaps form tightening or sealing edges and have the additional function of preventing liquid, and possibly faeces, from leaking-out at the edges, in other words they form barriers.

As an added safeguard against the leakage of liquid from the side-extremities of an article, a number of absorbent articles also include additional, inner liquid-barriers or cuffs fastened in the proximity of the longitudinal extremities of the article concerned. The purpose of the liquid barrier is to prevent liquid from leaking out at the edges of the absorbent article, and possibly also to prevent the escape of solids, such as faeces. This second or additional liquid barrier is placed inwardly of the leg elastic, i.e. inwardly of the outer liquid barrier, and is comprised of an essentially liquid-impermeable material, for instance a nonwoven material. The liquid barrier comprises a web of material whose one longitudinally extending edge is attached to the absorbent article and the other, free-edge of which is intended to lie against the wearer's skin. The free edge is elastically puckered or gathered together with the aid of an elastic element, such as a rubber, thread that is folded-in in the edge of the web. When the leg elastic on the absorbent article is stretched and the article fastened on the wearer, the elastic element of the liquid barriers will also be stretched, thereby raising the barriers up. The elastic element of the barriers will hold the barrier edges under tension against the wearer. Examples of diapers that include liquid barriers are found, for instance, in SE-T3-0 264 238 and GB-A-2 188 532.

WO-A1- 9319711 describes a sanitary napkin having barrier means located along the longitudinal sides of the sanitary napkin. The barrier means can be formed of a unitary part of the napkin's topsheet or can be formed of a separate strip of material. The barrier includes a contraction member which is connected in the form of an elastic.

EP-A1-0 534 488 describes sealing gaskets which are preformed to extend outward from the central part of an absorbent article, which is illustrated by a sanitary napkin in the description of the preferred embodiment. The gaskets may be formed by looping a strip of material, such as a non-woven material, so as to form a compliant cuff which bears against the user's body in a comfortable manner. In one embodiment the gaskets are attached directly to the extremities of the absorbent article, one edge of the gasket strip is attached to the upper sheet of the absorbent article and the other edge to the lower sheet. The looping of the material form cavitities for imparting compliancy and stability to the gaskets and the looping may also enclose elastic elements that are placed in tension when applied to the article so as to impart an arcuate shape to the article.

In another embodiment the gasket material is an elastic material which is attached to the longitudinally extremities of the sanitary napkin. The material is placed in tension when applied to the sanitary napkin. The purpose of doing so is to impart an arcuate shape to the article.

The sanitary napkin is placed in a panty crotch during use and is pressed against the wearer by the force from the panty. Thus, the force applied from the sanitary napkin against the wearer is indirectly caused by pressure from the panty. The gasket embodiments including elastic elements do not cause the force against the body of the wearer. The purpose of the elastic elements are, if they are used, to impart an arcuate shape. This differs from the diaper or incontinence guard according to the invention with a barrier element including an elastic device, wherein the barrier element will get stretched against the wearer during use and thereby creating a liquid barrier with a good sealing effect. It is the body of the user which makes the elastic device in the barrier to get tensioned.

Another type of barrier in an absorbent article is described in EP-A2-0 357 298. This article includes a bottom liquid-impermeable sheet, or backing sheet, an absorbent layer which is covered by a liquid-permeable casing, and an upper, or top, liquid-permeable sheet that is intended to lie proximal to the wearer in use. The shape and size of the upper sheet are roughly the same as the shape and size of the bottom sheet, and the upper sheet includes an aperture through which faeces and possibly urine can pass. The opening is formed in the sheet so that it will be located in the vicinity of the anus when the article is in use. A faeces accommodating pocket is formed between the upper sheet and the absorbent layer. The upper sheet has a band or ribbon of elastic material fastened longitudinally along the absorbent article, so as to cause the edges of said aperture to seal between the pocket and the wearer and to prevent faeces from coming into contact with the wearer's skin.

In the case of diapers that include liquid barriers or cuffs, the barrier will be positioned so as to lie against the wearer's thighs and buttocks, such as to prevent leakage in these areas. Each urine discharge will deliver a certain volume of liquid to the article. Because of the delay before this urine is able to penetrate through the upper sheet and be absorbed in the absorbent layer, a certain amount of urine will "float" in the absorbent article, on top of the surface sheet. It is this volume of urine that the barrier is intended to contain at the extremities of the absorbent article. Cuffs can also be used on sanitary napkins, for instance.

It has been found, however, that this barrier is not completely proof against leakage and that liquid is able to escape at the elastically puckered edge that lies against the wearer's skin. Because the barrier edge that lies against the wearer's skin is puckered, there form at the contact surface between the barrier and the wearer's skin small through-penetrating pores or passageways through which liquid is able to escape. If the barrier is extended, i.e. stretched, to a greater extent, the barrier will be puckered to a lesser degree. As a result, the through-passing pores will be smaller and a higher liquid pressure, breakthrough pressure, will be required before the barrier begins to leak. The barrier will, however, then be stretched hard against the wearer's thighs, which causes discomfort and reduces flexibility as the wearer moves.

The object of the present invention is to solve this problem.

### SUMMARY OF THE INVENTION

The present invention relates to a web-like element that includes an elongated, essentially non-elastic material and an elongated, web-like elastic device having two longitudinally extending edges, wherein the elastic device is attached to the essentially non-elastic material such that a longitudinal part of the elastic device is free. The web-like element is intended for use as a liquid barrier in an absorbent article, and is attached to the essentially inelastic material whilst in a stretched state, so as to pucker said inelastic material. The free part of the elastic device will suitably have a generally smooth surface both in a relaxed and a stretched state.

The invention also relates to an absorbent article that includes longitudinally extending and transversely extending extremities, a bottom liquid-impermeable sheet, an absorbent layer, an upper liquid-permeable sheet which is intended to lie proximal to the wearer in use, and on each side of the centre line of the upper sheet at least one longitudinally extending liquid barrier which is comprised of a substantially liquid-impermeable material and which is attached along or adjacent to the longitudinal extremities of the article and includes a free, stretchable edge that is intended to lie proximal to the wearer in use, wherein the liquid barrier includes an elongated, web-like elastic device that has two longitudinally extending edges and includes a longitudinally extending free part which forms the stretchable edge and which is intended to lie against the wearer in use. The liquid barriers are attached along or adjacent to the longitudinal extremities of the article, and optionally also adjacent the two transversal extremities thereof.

The invention also relates to an absorbent article that includes a bottom liquid-impermeable sheet which is intended to lie distal from the wearer in use, an absorbent layer, a liquid-permeable sheet disposed on top of said absorbent layer, and an upper liquid-impermeable sheet which is intended to lie against the wearer in use and which includes elastic for shaping the article to the shape of the wearer's body. The upper sheet includes at least one aperture for location in register with the anus and the urethra orifice of the wearer. The upper liquid-impermeable sheet also includes liquid barriers attached around said aperture or apertures in the aforedescribed manner.

The invention also relates to the use of an inventive element as a liquid barrier in absorbent articles.

### DETAILED DESCRIPTION OF THE INVENTION

The invention will now be described in more detail with reference to particular nonlimiting embodiments thereof and also with reference to the accompanying drawings, in which

Fig. 1 illustrates a diaper that includes conventional liquid barriers.

Fig. 1a is an enlarged, exploded cross-sectional view of the part I in Fig. 1 of a typical web-like element.

Figs. 1b, c, d, e, f and g illustrate different embodiments of an inventive web-like element, corresponding to an enlarged cross-sectional view of the part I in Fig. 1.

Fig. 2 is a comparison diagram of respective calculated and measured breakthrough pressures at different available elongation or stretch factors of a conventional liquid barrier.

Fig. 3 illustrates how a liquid barrier in the absorbent article will lie against the skin of the wearer, in accordance with one embodiment of the invention.

Fig. 4 is a photograph of a three-ply film stretched to 150%, said photograph being taken with a scanning electron microscope and enlarged 300 times.

Figs. 5 and 6 are photographs of one embodiment of an inventive liquid barrier taken with a scanning electron microscope, there being used a three-ply film similar to that used in the photograph shown in Fig. 4. The liquid barrier lies against Plexiglas at a respective available elongation of 50 and 0%. The photographs are taken with a 5% inclination to the Plexiglas.

Figs. 7a, 7b illustrate measuring equipment used to determine the leakage pressure or breakthrough pressure.

Fig. 7c is an outline view of a measuring process using the equipment shown in Figs. 7a, b.

Fig. 8 illustrates schematically a pore formed between a liquid barrier and Plexiglas, and illustrates the principle of determining the radius r.

Figs. 9a, 9b illustrate the principle in which available elongation or stretch is calculated.

Figs. A-E are photographs taken with a scanning electron microscope of a conventional liquid barrier from a diaper produced by Kimberly-Clark and designated Huggies, with the standing gather resting against curved Plexiglas. The photographs are taken at an available elongation of 10, 20, 30, 40 and 50% respectively.

Figs. Aa-Ea are views corresponding to the photographs in Figs. A-E.

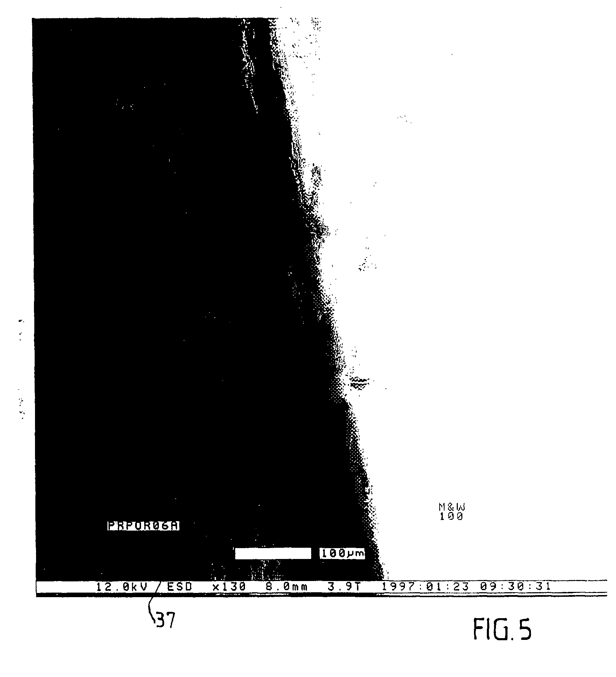

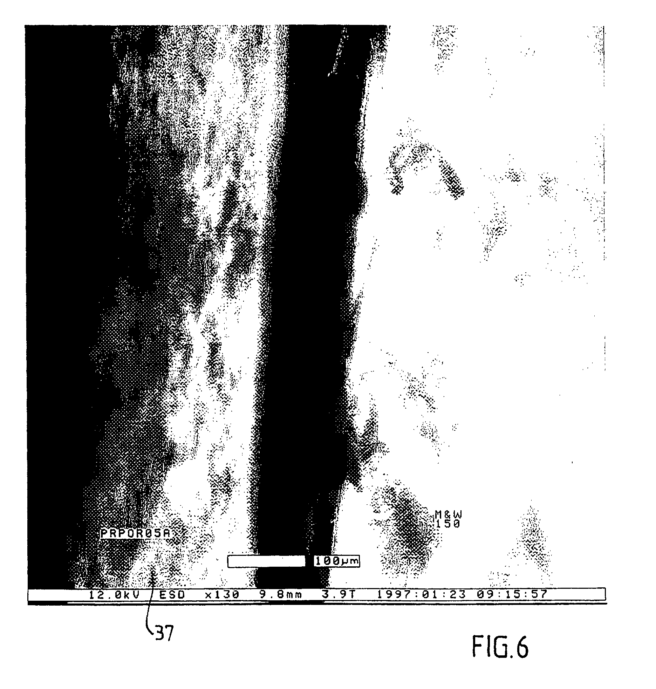

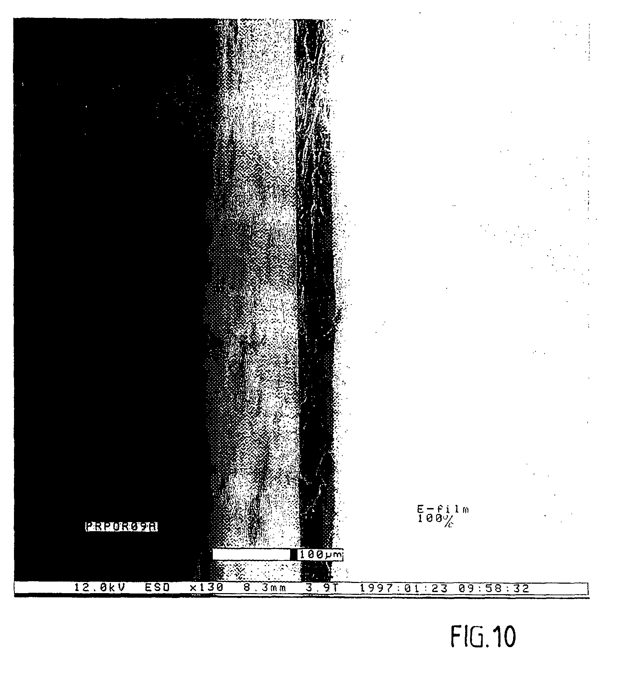

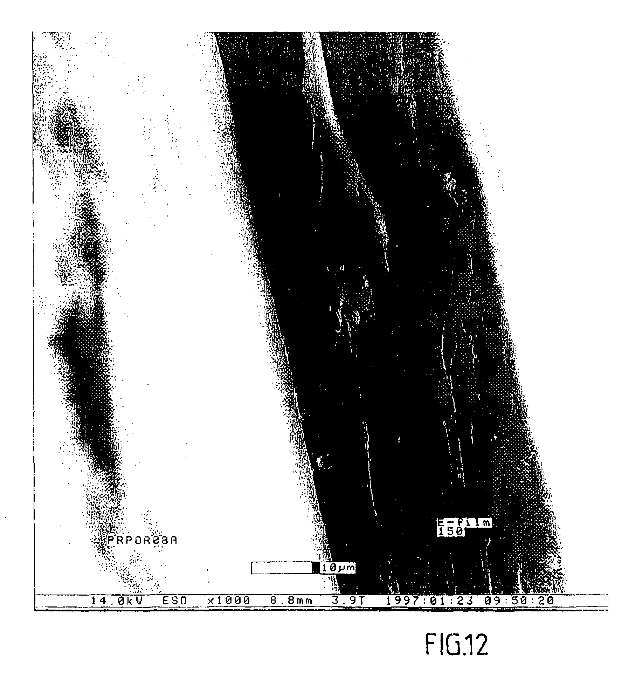

Figs. 10-12 are photographs taken with a scanning electron microscope of one embodiment of an inventive liquid barrier in which elastic film was used. The photographs correspond to the photographs shown in Figs. 5 and 6 and are enlarged 130 times with respect to Figs. 10, 11 and 1000 times in respect of Fig. 12.

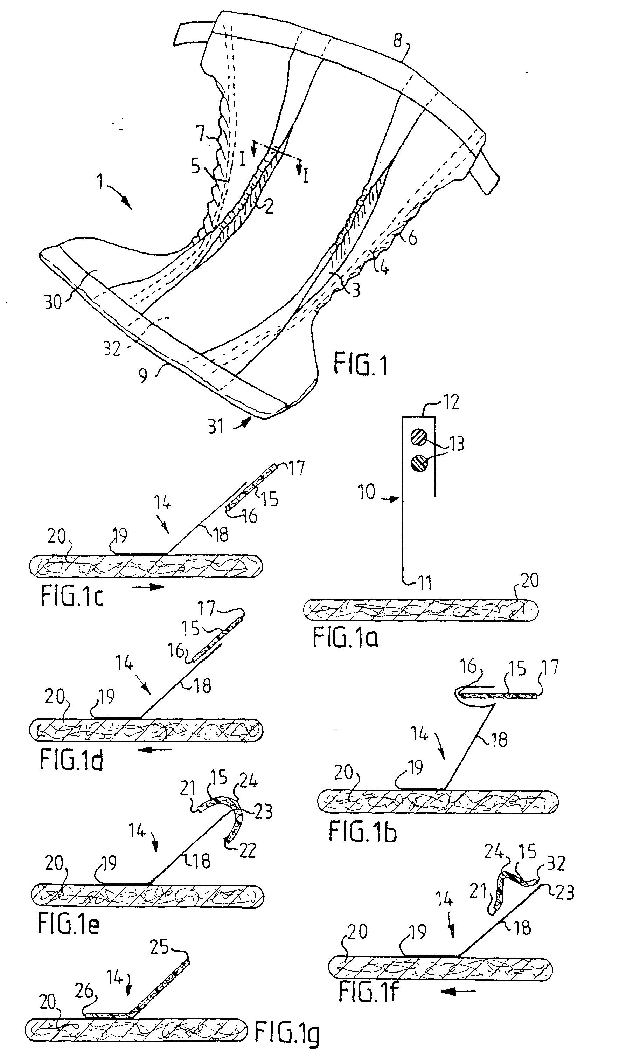

Figs. 1 and 1a illustrate a typical diaper or incontinence guard 1 that includes an upper liquid-permeable sheet 30, an absorbent layer 32, and a bottom liquid-impermeable sheet 31, said sheets being delimited by two transverse edges 8, 9 and two longitudinal edges 6, 7. A respective leg elastic 4, 5 extends longitudinally along each side of the longitudinally extending centre line of an upstanding liquid barrier 2, 3.

The diaper shown in Fig. 1 thus typically includes inner liquid barriers 2, 3 and outer barriers constituted by the leg elastic 4, 5. The outer barriers, i.e. the leg elastic 4, 5, are, as a rule, comprised of elastic threads which are fastened in a stretched state between the top and bottom sheets 30, 31, of the absorbent article. The liquid barriers 2, 3 are positioned in the proximity of the two longitudinal extremities 6, 7 of the article 1. Each inner barrier 2, 3 is comprised of a web-like element 10 made of an essentially liquid-impermeable material and one longitudinal edge 11 is attached to the liquid-permeable upper sheet 30 or to the liquid-impermeable bottom sheet 31 in the proximity of the longitudinal extremities extremities 6, 7. The other, free edge 12 of the element (Fig. 1a) is intended to lie against the wearer of the article and is puckered elastically with the aid of an elastic thread or threads 13 fastened in a stretched state in an inwardly folded part of the barrier element. This will be evident from Fig. 1a, which is an enlarged cross-sectional view of a typical barrier 2, 3. The sheets are shown separated from one another, for the sake of clarity. When the diaper is in use, the liquid barriers 2, 3 will lie against the crotch and buttocks of the wearer and prevent the escape of liquid in these areas. Elastic puckering or gathering of the material, however, causes through-passing pores or passageways to form in the contact surface between carrier and the user's skin. Such pores or passageways are also formed at the leg elastic.

By "pores" is meant the through-penetrating passageways that are formed between a liquid barrier and the wearer's skin and through which liquid can pass. These pores are formed mainly because the liquid barrier is puckered where it lies against the wearer's skin. The more the barrier is relaxed, the larger the pores that are formed.

The expression "available elongation" is used with respect to the extent to which a liquid barrier can be stretched or extended.

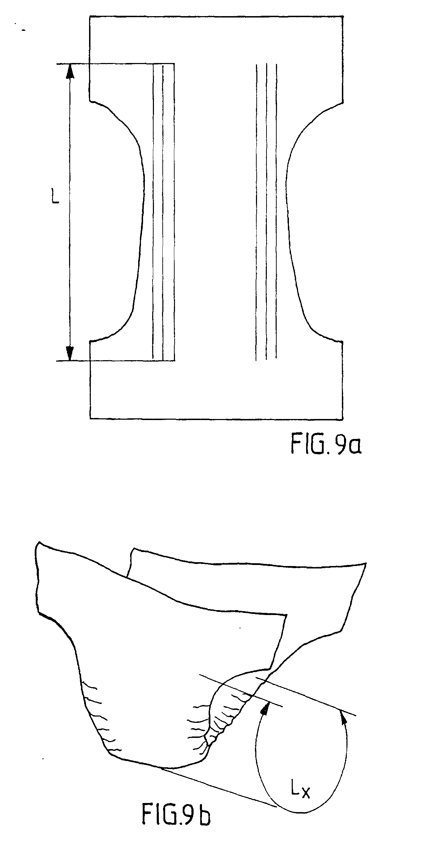

In the manufacture of the absorbent article, e.g. a diaper, the elastic material, which has a given degree of stretchability, is "locked" firmly to remaining non-stretchable material, normally nonwoven. The extent to which the elastic material is stretched in the manufacture of the article cannot be exceeded when the article is in use, since the elastic material is firmly secured to a non-stretchable material. This is shown in Fig. 9a. The elastic material has the length **L.**

A diaper is puckered somewhat when placed on the wearer's body. The elastic material has then contracted to the smaller length **L**_{**x**}**,** shown in Fig. 9b. The available stretch or elongation **X** is the extent to which the material can be stretched from the user state to the maximum stretched state of the product. This can be expressed by the formula: **L** = **L**_{**x**} **(X/100)** + **1),** where **X** is the available stretch or elongation in percent. **L**_{**x**} is given by the size of the wearer, **L** is obtained from the product specification. This enables the available stretch **X** to be calculated.

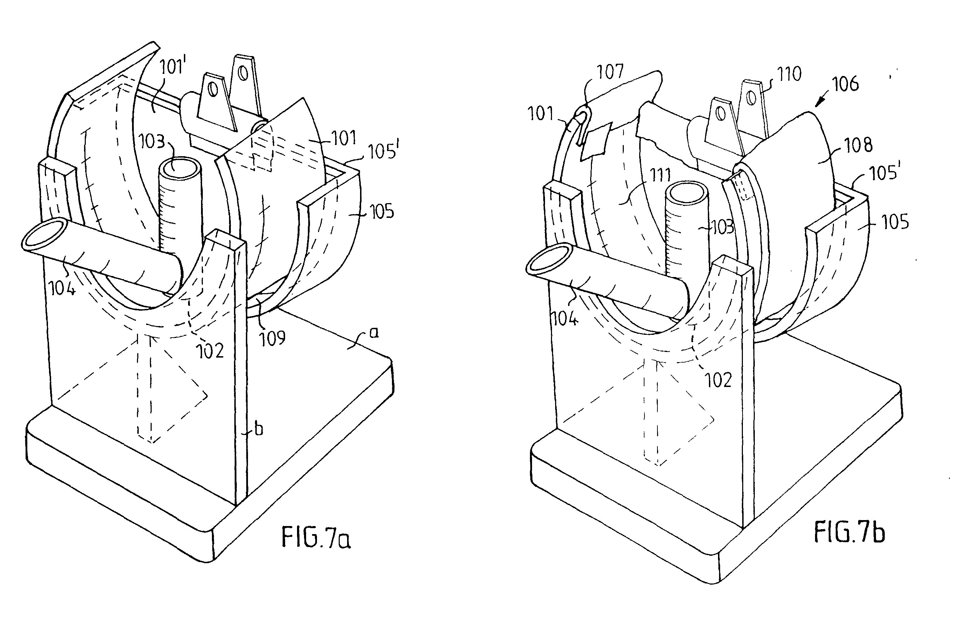

Test equipment was constructed with the intention of studying the sealing effect achieved between a liquid barrier or some other puckered barrier and the wearer's skin. This equipment is shown in Figs. 7a, 7b and 7c and comprises a Plexiglas stand which includes a base plate a and an upstanding support plate b. A first upwardly open, semi-cylindrical element 101 is fastened horizontally to the upstanding support plate b and has around its periphery a scale which denotes the available elongation or stretch. One end of the semi-cylindrical element is attached to the support plate while the other end has an end-wall 101'. Provided at the very bottom of the semi-cylindrical element 101 is a hole 102 to which a vertically upstanding filling tube 103 and an inclined measuring tube 104 lead, both of said tubes having a scale expressed in mm water. The equipment also includes a loose second semi-cylindrical element 105 whose diameter is somewhat larger than the diameter of the first semi-cylindrical element 101 and which has one side open and an end-wall 105' at its other end.

As shown in Fig. 7b, a measuring operation is carried out by stretching and securing a liquid barrier around the outer periphery of the first semi-cylindrical element and fastening said barrier around the upper edges. The elastic part 107 is directed towards the attachment of the semi-cylindrical element to the carrier plate b, and the liquid barrier material is folded around the end-wall 101' of the first semi-cylindrical element 101 on the other side. The elastic part is fastened along the scale on the semi-cylindrical element so as to enable the available elongation or stretch to be read-off. The end-wall 105' of the second semi-cylindrical element 105 is placed against the end-wall 101' of the first semi-cylindrical element with said upfolded part of said barrier material located therebetween and pressed thereagainst with the aid of a clamp 1010, such as to obtain a small clearance 109 between the cylindrical walls. Synthetic urine is introduced through the vertical tube 103. The liquid barrier is first weighted down so as to fill the clearance between the semi-cylindrical elements. A liquid pressure is thereafter built-up against the elastic edge 107 at the same time as a liquid column is formed in the tubes 103, 104, where the pressure can be read-off. Liquid is introduced until leakage occurs at arrow B (Fig. 7c) at the breakthrough pressure.

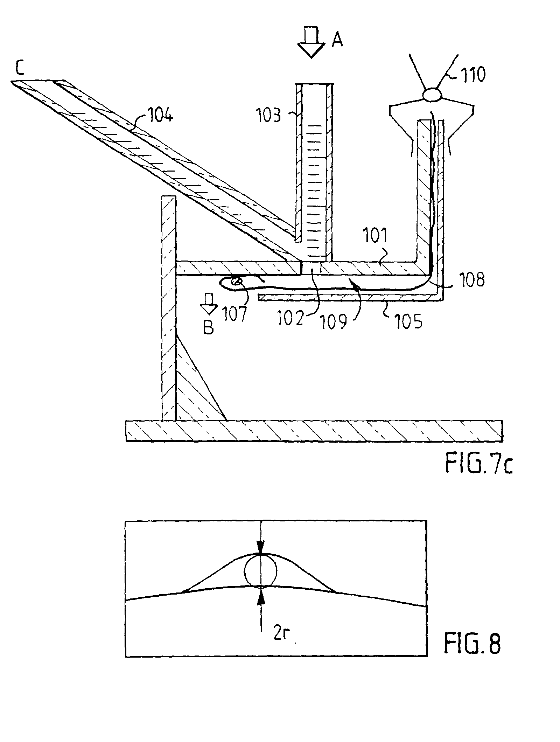

As illustrated in Figs. A-E and Aa-Ea, abutment of a liquid barrier with the illustrated measuring equipment has been studied with an electron microscope, enlargement 130 times. The liquid barrier used was a conventional liquid barrier taken from a diaper manufactured by Kimberly-Clark and designated Huggies. The Plexiglas stand corresponds to the wearer of the diaper in the illustrated case. The standing gather has been stretched to different extents, to different available elongations, therewith forming pores 30 of mutually different sizes. The pores 30 are seen as the dark areas between the light Plexiglas 31 on the left edge and the fibres 34 of the barrier 33. The pores are assumed to function as capillaries, wherewith r equals the radius of the largest possible circle 32 that can be enclosed therein. Fig. 8 shows the principle on which the pore radius is determined.

The through-penetrating pores 30 formed between the threads or the fibres 34 in the barrier material 33 and the Plexiglas 31 have been drawn in Figs. Aa-Ea. The largest possible circle that can be enclosed in the pores and that determines the pore radius is also shown. The following values were obtained in respect of pore radius at different available elongations, as shown in the Figures.

| **Available elongation** | **Pore radius** |
|---|---|
| 10% | 0.0208 mm |
| 20% | 0.0812 mm |
| 30% | 0.1208 mm |
| 40% | 0.1458 mm |
| 50% | 0.1458 mm |

Comments: It was very difficult to measure the pore size on the photograph at an available elongation of 10%, and the 10% value is therefore perhaps unreliable.

A study of the photographs reproduced in Figs. Aa-Ea shows that the pores 30 between the Plexiglas 31 and the liquid barrier 33 become larger with higher available elongations. Thus, the more relaxed the liquid barrier, the larger the pores. This means that the barrier will leak at a lower pressure with higher degrees of barrier relaxation, and with higher degrees of stretch at higher breakthrough pressures.

The same diaper will leak at different rates, depending on the size of the wearer. When the diaper is worn by a large person with thick thighs, the leg elastic and the inner barriers will be relatively tight and the pores smaller than when, e.g., a smaller or more slender person with thinner legs uses the diaper. In this latter case, the barrier will sit loosely around the wearer's thighs and in the wearer's crotch and the pores will therewith be larger than in the former case, resulting in a higher leakage risk because of the larger pore size and because of the lower tension in the elastic. The tension in the barrier will also influence the way in which the diaper fits around the wearer's body.

Because the elastic device in the inventive web-like element has a substantially smooth surface irrespective of whether it is stretched or relaxed, those pores that are formed in the contact surface between the liquid barrier and the wearer's skin will have a smaller radius (according to the definition above) than the pores of a conventional liquid barrier. Furthermore, the pore radius will remain essentially unchanged as the barrier is tensioned or relaxed. Because the pore radius is smaller, it is more difficult for liquid to leak out at the contact surface between the inventive barrier element and the wearer's skin and the breakthrough pressure increases.

Since the pore radius will remain essentially unchanged whether the barrier is tensioned or relaxed, the same size of diaper can be used by persons of mutually different sizes, without changing the pore radius formed in the contact surface between the liquid barrier and the wearer's skin.

The invention will now be described in more detail with reference to a number of non-limiting, exemplifying embodiments.

The invention concerns a web-like element 14 intended for use as a liquid barrier in absorbent articles 1, 20, 28, such as diapers, incontinence guards or like articles, wherein the element 14 includes an elongated essentially inelastic and essentially liquid-impermeable material 10, 18, and an elongated elastic device 13, 15 attached in a stretched state to the material 10, 18 such as to pucker said material, wherein the elastic device is a band-like device 15 having two longitudinally extending edges 16, 17, 21, 22 and fastened to the material 18 such that a longitudinally extending part 17, 24 of the elastic device 15 remains free.

The invention also concerns an absorbent article 1, 20, 28 such as a diaper, an incontinence guard or like article, that includes longitudinally extending 6, 7 and transversely extending 8, 9 extremities, a bottom liquid-impermeable sheet 31, an absorbent sheet 32, an upper liquid-permeable sheet 30, wherein the articles includes at least one longitudinally extending liquid barrier 2, 3, 4, 5 comprised of essentially liquid-impermeable material 10 and fastened along or adjacent to the longitudinal extremities 6, 7 of the article on each side of the centre of the top sheet, said liquid barrier including a free, stretchable edge 12 ,17, 24, 25 intended to face towards the wearer in use, or the article include an upper liquid-impermeable sheet which is intended to lie against the wearer and which includes elastic for shaping the article to the wearer's body, wherewith the upper liquid-impermeable sheet is fastened to the bottom liquid-impermeable sheet at its outer extremities 6, 7, 8, 9 and wherewith the upper sheet includes at least one aperture for register with the anus and urethra orifice of a wearer, and wherewith the upper liquid-impermeable sheet includes elastic liquid barriers comprised of essentially liquid-impermeable material fastened around said aperture or apertures, wherein the liquid barrier includes an elongated, band-like elastic device 15 that has two longitudinally extending edges 16, 17, 21, 22 and includes a longitudinally extending free part 17, 24, 25 that forms the stretchable edge intended to lie against the wearer in use.

### Examples

Shown in Fig. 1 is an absorbent article constructed in accordance with one embodiment of the invention, such as a diaper, sanitary napkin, incontinence guard or like article, preferably diaper or incontinence guard. The absorbent article has longitudinally and transversely extending extremities 6, 7, 8, 9 and includes a liquid-impermeable sheet 31, an absorbent layer 32, an upper liquid-permeable sheet 30 that is intended to lie against the wearer's body in use, and at least one longitudinally extending liquid barrier 2, 3, 4, 5 produced from an element 14 comprised of essentially liquid-impermeable material attached along or adjacent the longitudinally extending extremities 6, 7 of the article on each side of the longitudinal centre line of the upper sheet. When the article is a diaper, for instance, it will include a waist-part at its front and rear parts, and an intermediate crotch-part. Its transverse extremities 8, 9 will be placed around the wearer's waist and the longitudinal extremities 6, 7 placed around the wearer's crotch and thighs when the article is donned.

In one embodiment, the leg elastic devices 4, 5 constitute the outer liquid barriers and the inner cuffs 2, 3 constitute the inner liquid barriers 2, 3. In another embodiment of the invention, the leg elastic devices are the sole liquid barriers 4, 5.

As mentioned the liquid barrier includes an elongated, web-like elastic device 15 that has two longitudinally extending edges 16, 17, 21, 22 and a longitudinally extending free part 17, 24, 25 that is intended to lie against the wearer. The liquid barrier may also include an elongated, web-like, essentially inelastic material 18 that has two longitudinally extending edges 19, 23, where one edge 19 is attached to the upper liquid-permeable sheet 30 of the absorbent article 1, 20, 28 or to its bottom liquid-impermeable sheet 31, while the second edge 23 is attached to the elastic device 15. The free part 17, 24, 25 of the elastic device has a substantially smooth surface in both a relaxed and a tensioned state, and the pore size will mainly be independent of the available elongation or stretch. In some embodiments (see Figs. le and 1f), the web-like elastic device is attached to the inelastic material 18 along both its longitudinal edges 21, 22 and forms a fold or bend 24. In these embodiments, it is the fold or the bend 24 that forms the free longitudinal part that is intended to lie against the wearer in use.

The elements 14 shown in Figs. 1b-g can all conceivable be used in an inventive absorbent article; they are all different embodiments of the element used as liquid barriers in the absorbent article. In the embodiment shown in Fig. 1g, the elastic device is fastened to the absorbent article 20 in a stretched state, whereas in the embodiments shown in Figs. 1b-f, the elastic device 15 is fastened to the inelastic material 18 in a stretched state. The elements 14 are used as liquid barriers at the leg elastic 4, 5 or at the inner liquid barriers, the cuffs 2, 3. They can also be used at both the leg elastic and the cuffs, although they are preferably used at the innermost liquid barriers. When the absorbent article has no cuffs, the leg elastic will thus constitute the innermost liquid barriers. The liquid barrier will provide a seal with small pores, irrespective of the extent of the available elongation or stretch. The pores will have a radius of at most 0.1 mm, suitably at most 0.05 mm, and most preferably 0.02 mm.

Fig. 1b is an enlarged sectional view of one embodiment of an inventive web-like element 14. An elongated web-like elastic device 15 having two longitudinally extending edges 16, 17 is fastened in a stretched state with one edge 16 against an elongated, essentially inelastic liquid-tight material 18. The elastic device 15 is fastened in an inwardly folded portion of the essentially inelastic material 18. The other edge 17 of the elastic device is free and forms a longitudinally extending free portion of the elastic device. The free edge 19 of the elongated, essentially inelastic material is intended to be fastened to the absorbent article 20 in the proximity of its longitudinally extending extremities. The free edge 17 of the elastic device has a substantially smooth surface both in a relaxed and a stretched state and is intended to provide a "seal" in the contact surface between the wearer's skin and the liquid barrier. The elastic device 15 has a width of at least 0.5 cm, preferably at least 1.0cm.

Figs. 1c-g illustrate other embodiments of an inventive web-like element. Fig. 1c illustrates a web-like element 14 in which the one edge 16 of said elastic device 15 is fastened to the essentially inelastic material 18 on that side thereof which faces towards the centre of the absorbent article 20 in the arrowed direction. The free edge 17 is formed by one edge of the elastic device 15.

The embodiment illustrated in Fig. 1d is similar to that illustrated in Fig. 1c, with the exception that one edge 16 of the elastic device 15 is fastened to that side of the inelastic material 18 which faces outwards from the centre of the absorbent article in the arrowed direction. As with the embodiment shown in Fig. 1c, the free edge 17 is formed by one longitudinal edge of the elastic band 15.

Fig. 1e shows a web-like element 14 with which the elastic band-like device 15 is folded around one edge 23 of the inelastic material 18 and fastened thereagainst by the two longitudinal edges 21, 22. The elastic band-like device includes a fold or bend 24 in the region between said two edges, said bend not being fastened to the inelastic material 18. The bend 24 presents a smooth surface in both a relaxed and a tensioned state. In this embodiment, the fold or the bend 24 forms the aforesaid longitudinal free part of the elastic band-like material 15 intended to lie against the wearer in use. The longitudinal edges 21, 22 are shown separated from the elastic device for the sake of clarity.

Fig. 1f illustrates a web-like element 14 with which the two longitudinal edges 21, 22 of the band-like elastic device 15 are fastened to one side of the inelastic material 18, on the one hand against its edge 23 and on the other hand further down on that side which faces away from the centre of the absorbent article 20 (see the arrow). The band-like device is folded to form a fold or bend 24 in the region between the two edges 21, 22. As with the embodiment shown in Fig. le, it is this fold or bend 24 that forms the longitudinally extending free portion of the band-like elastic material 15 intended to lie against the wearer in use.

Fig. 1g shows another embodiment, in which the web-like element 14 consists totally of an elongated elastic device. The elastic device has two longitudinally extending edges 25, 26, where one edge 25 forms the longitudinally extending free portion of the elastic device and is intended to lie against the wearer in use, and the other edge 26 is intended to be fastened in a stretched state to the upper liquid-permeable sheet 30 or to the bottom liquid-impermeable sheet 31 of the absorbent article 20, in the vicinity of the longitudinally extending edges 6, 7.

In the case of the web-like elements shown in Figs. 1b-f, the elastic band-like devices 15 are fastened to the inelastic material 18 by, e.g., welding or gluing. The band-like elastic device 15 includes a longitudinally extending free part 17, 24, 25 which is intended to lie against the wearer in use. The elastic device is fastened in a stretched state to the web-like inelastic material 14, 18 in the embodiments shown in Figs. 1b-f, and to the upper liquid-permeable sheet 30 or the liquid-impermeable sheet 31 of the absorbent article in the embodiment shown in Fig. 1g. The elastic device has a substantially smooth surface both in a relaxed and a tensioned state. The barrier element of all embodiments includes a stretchable edge formed by the elastic device.

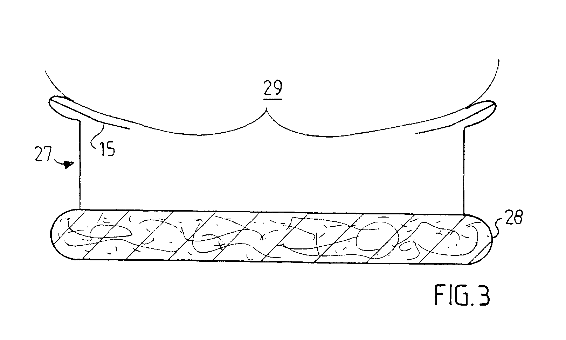

Because the elastic device 15 lies against the wearer's skin and has an essentially smooth surface, it will function as a "seal" between the wearer's skin and the liquid barrier. Fig. 3 is a schematic illustration of how the band-like elastic device 15 in the liquid barriers 27 of the absorbent article 28 lie against the wearer 29 in one of the embodiments. The element described with reference to Fig. 1b is the element used for the liquid barrier 27 in this embodiment.

When a barrier element according to the invention is used in an absorbent article such as a diaper or an incontinence guard, the body of the wearer directs the tensioning of the elastic device in the barrier element. Thus, the elastic is tensioned during use. This is necessary for the function of the barrier element in the absorbent article. The body of the wearer decides how much the elastic device will be tensioned. The elastic part will direct the barrier uppwards against the body and the body makes the barrier stretched during use. It is the tensioning from the body which makes the barrier sealing against the body. This differs from the sealing gasket described in EP-A1-0 534 488, wherein the elastic part of the gasket remain in the same tension irrespective of the size of the wearer. The elastic part does not raise the barrier, the main purpose for the elastic part in the gasket is to impart an arcuate form to the sanitary napkin. Besides, the elastic part is not necessary in the gasket. The force applied from the sanitary napkin against the wearer is indirectly caused by pressure from the panty, in which the sanitary napkin is placed. Also, the gasket will remain in the same tension irrespective of the size of the wearer.

In a further embodiment, a second barrier is fastened adjacent each transverse extremity 8, 9 of the absorbent product.

According to a second embodiment of the absorbent article of the invention, the article includes a bottom liquid-impermeable sheet which is intended to lie distal from the wearer in use, an absorbent layer, a liquid-permeable sheet disposed on said absorbent sheet, and an upper liquid-impermeable sheet which is intended to lie against the wearer in use and which includes elastic for shaping the absorbent article to the wearer's body. The upper liquid-impermeable sheet is fastened to the bottom liquid-impermeable sheet at their respective outer extremities, and this upper sheet includes at least one hole for location in the vicinity of the anus and the urethra orifice of the person wearing said article. The upper liquid-impermeable sheet also includes elastic liquid barriers formed from an element 14 which comprises essentially liquid-impermeable material and which is fastened around said aperture or apertures. The liquid barrier is formed in the same way as the liquid barrier used in the aforedescribed article and the embodiments illustrated in Figs. 1b-g can also be used in this second embodiment of the invention.

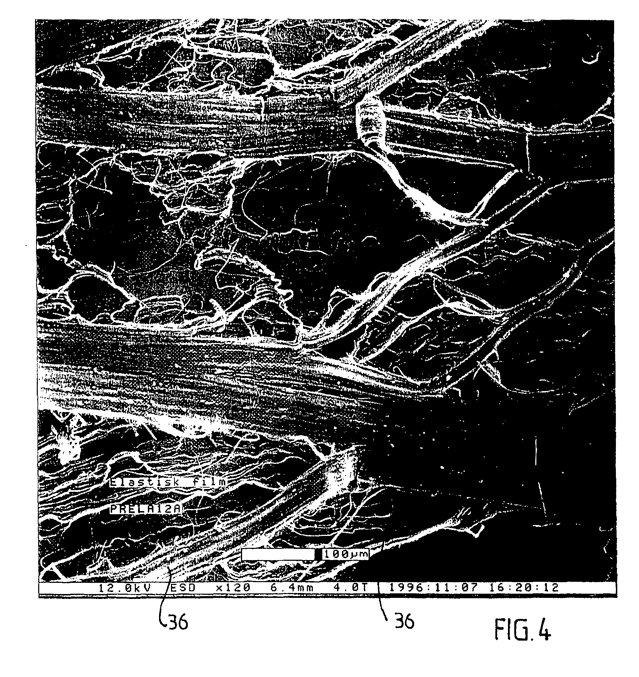

Figs. 4-6 are reproductions of photographs taken with a scanning electron microscope of a three-ply film used as the band-like elastic device in one embodiment of the invention. The photographs were taken with an enlargement of 120 times with respect to Fig. 4 and with an enlargement of 130 times with respect to Figs. 5 and 6.

Fig. 4 shows a photograph of a three-ply film stretched to 150%, and taken from above. The three-ply film is comprised of an elastic inner layer of styrenebutadiene-styrene (SBS) and two inelastic outer layers or plies of polypropylene (PP). When the three-ply film is stretched, the PP film breaks-up to form a network of threads and bands. The crackled PP film is seen as light regions 35 and band-like regions 36.

Figs. 5 and 6 are photographs of the liquid barrier with an available elongation or stretch of 50% and 0% respectively when lying against Plexiglas 37. The three-ply film has been stretched to 150% and secured at this elongation, thus corresponding to an available elongation or stretch of 0%. An available elongation or stretch of 50% (Fig. 5) means that the film has been stretched to 100%.

In this case, the film lies relatively tightly against the Plexiglas 37. The pores formed are elongated, with a small height. The pore radius is about 0.02 mm in both Fig. 5 and Fig. 6. The visible "layers" 38 are the band-like regions 36 in Fig. 4.

In this embodiment of the invention, the pore radius is independent of the available elongation or stretch. The pore radius is about 0.02 mm, which corresponds to the pore radius obtained with a conventional barrier with a 10% available elongation.

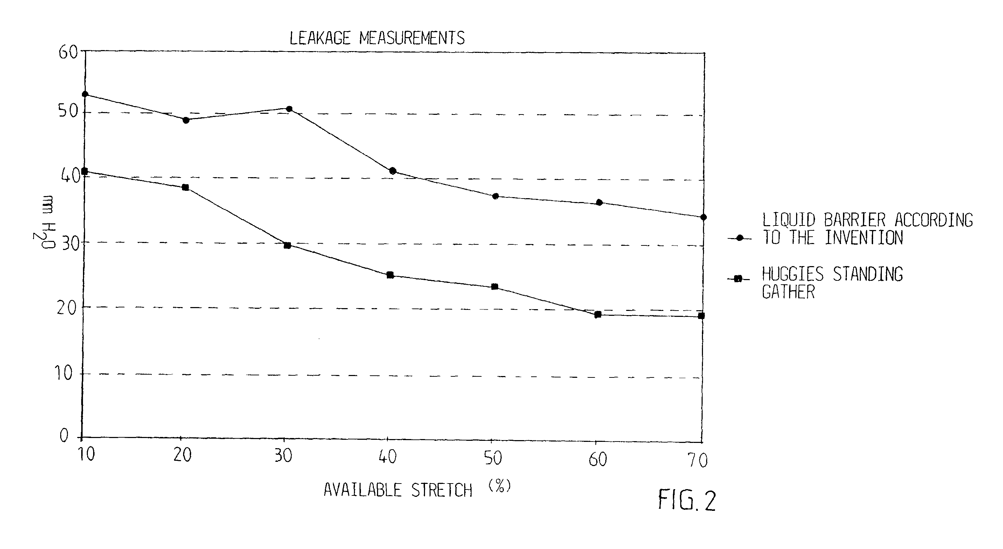

Fig. 2 is a diagrammatic illustration of the breakthrough pressures measured at different available elongations in respect of this embodiment of the invention and, by way of comparison, in respect of the liquid barrier illustrated in Figs. A-E. It will be evident from the diagram that much higher breakthrough pressures are obtained with the present liquid barrier than with the conventional liquid barrier.

Despite the fact that the pore radius of the illustrated barrier is essentially constant at different available elongations, the breakthrough pressure increases with decreasing available elongation. This is thought to be due to the increased tension in the elastic.

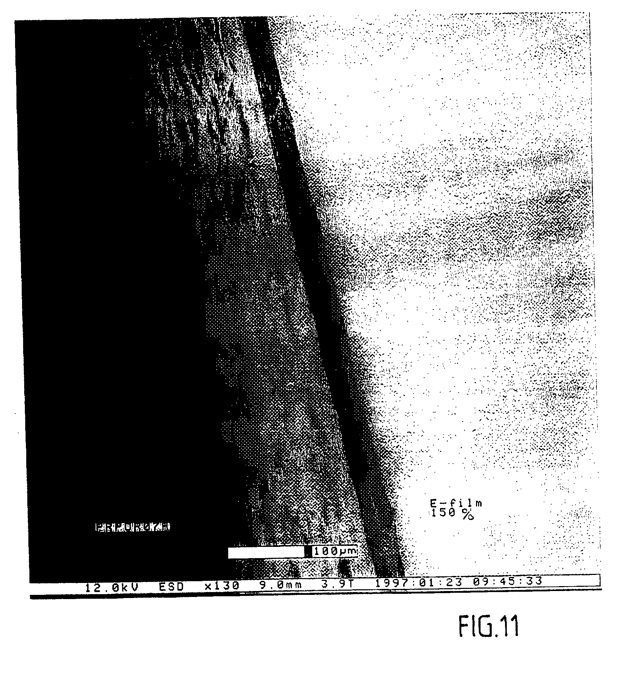

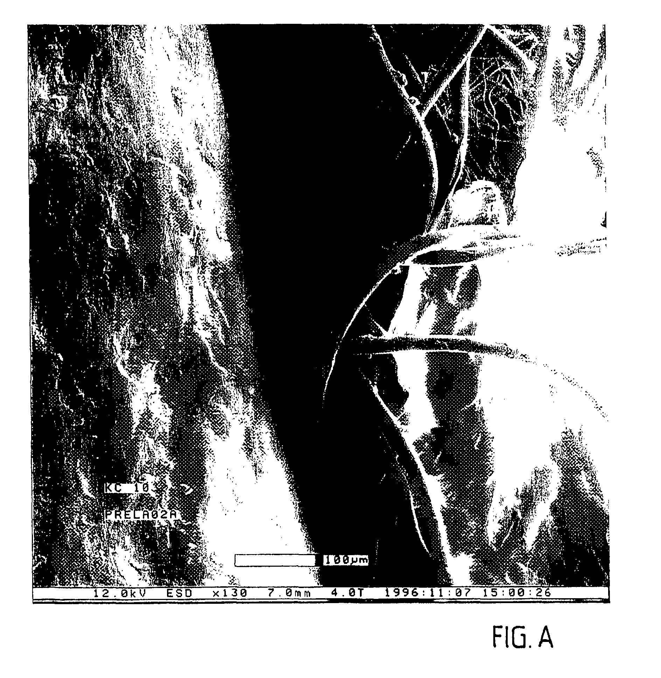

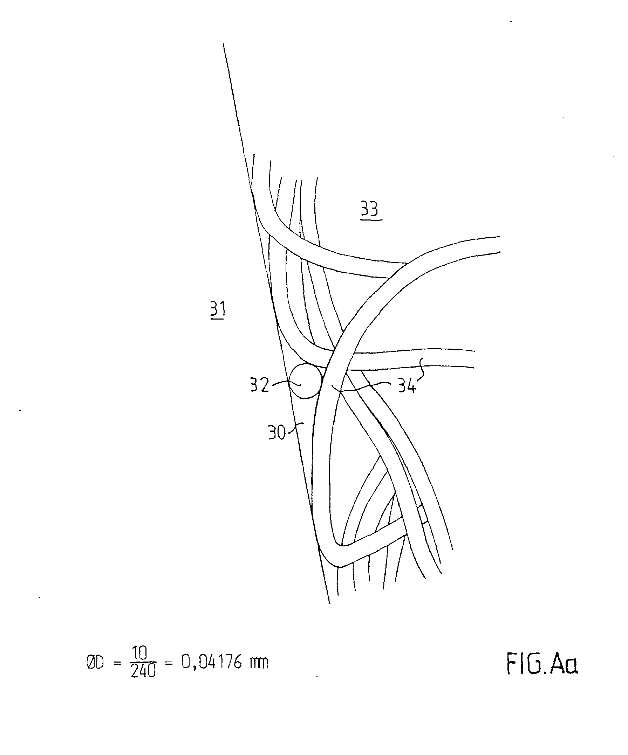

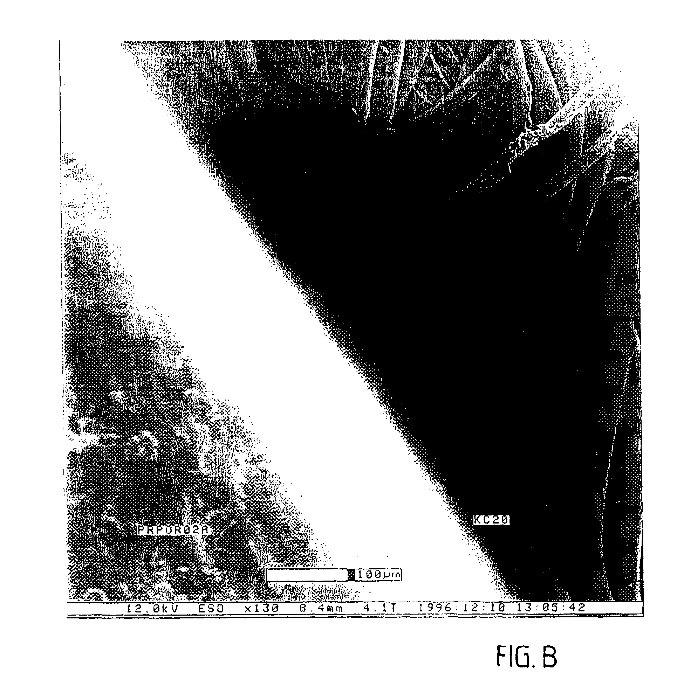

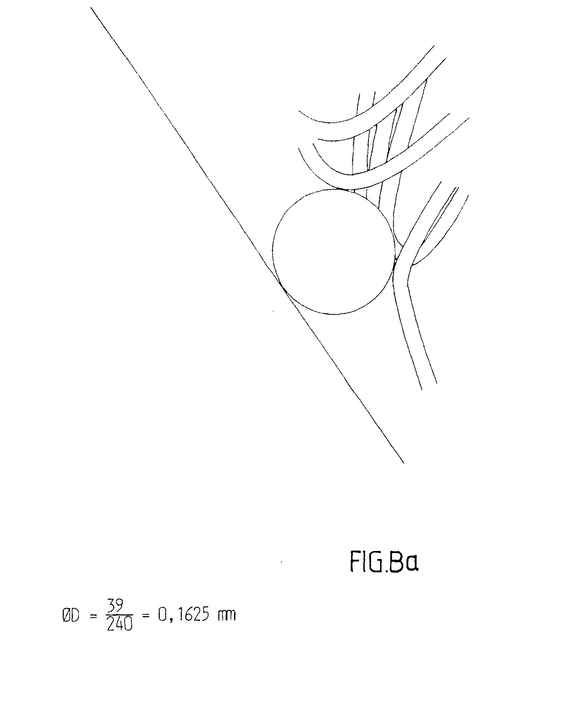

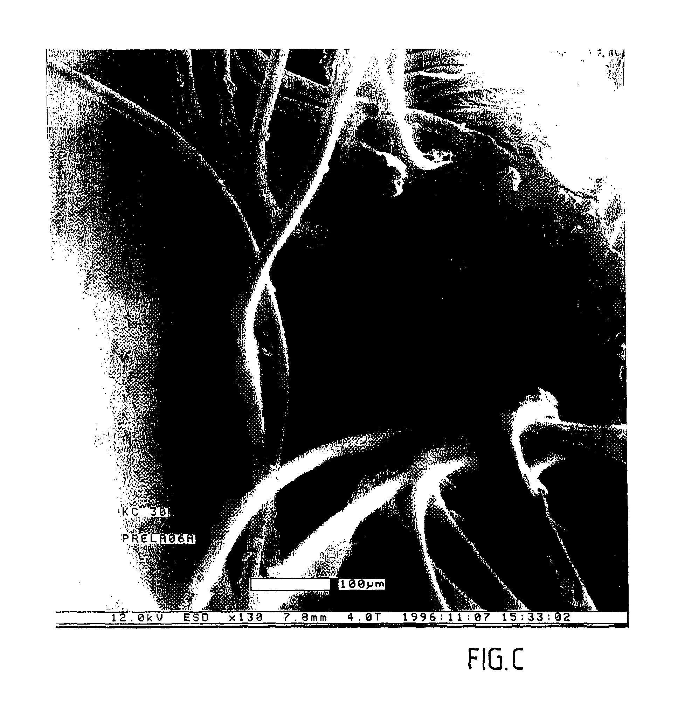

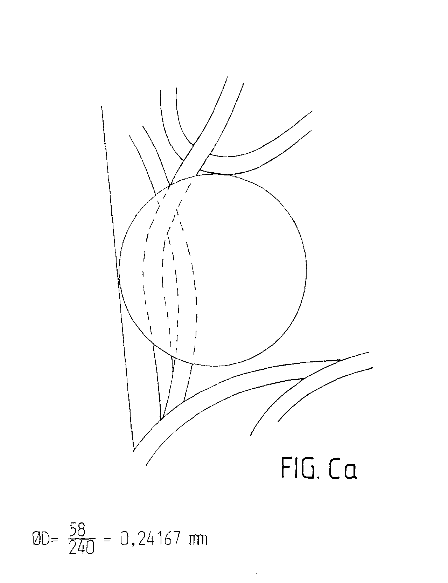

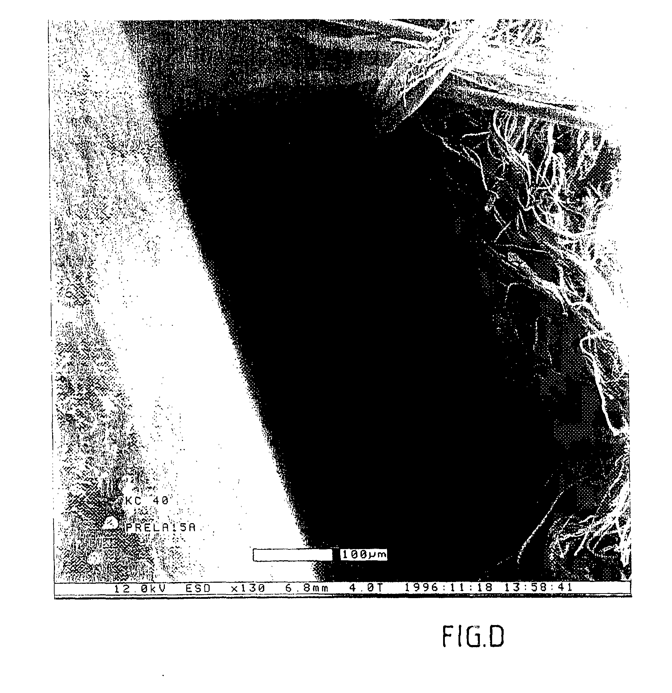

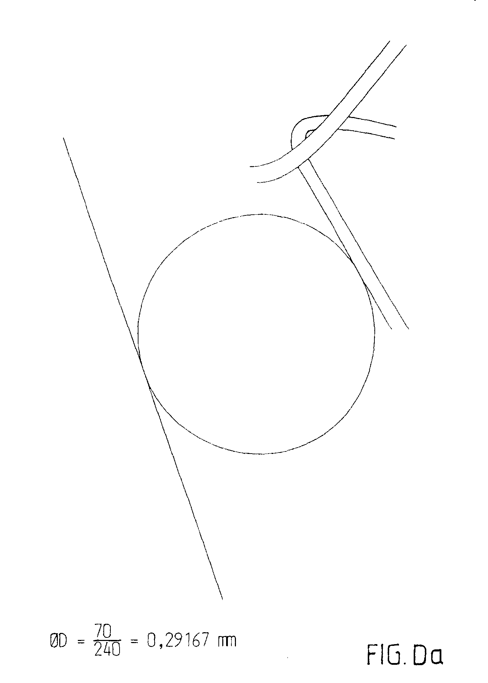

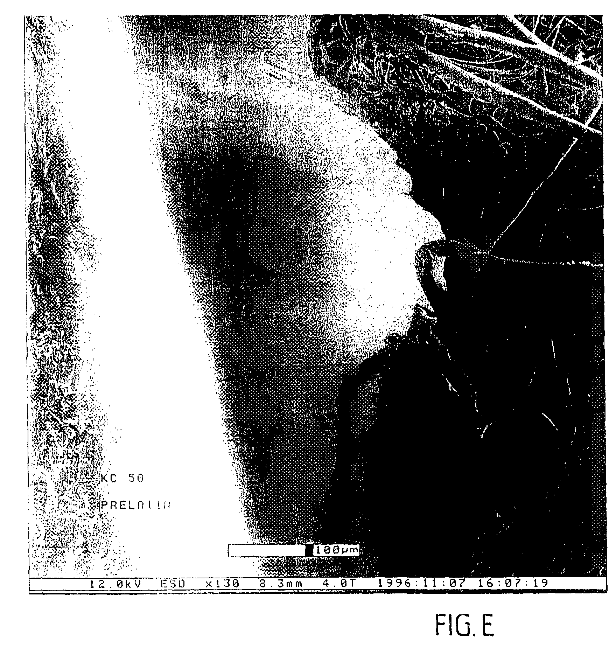

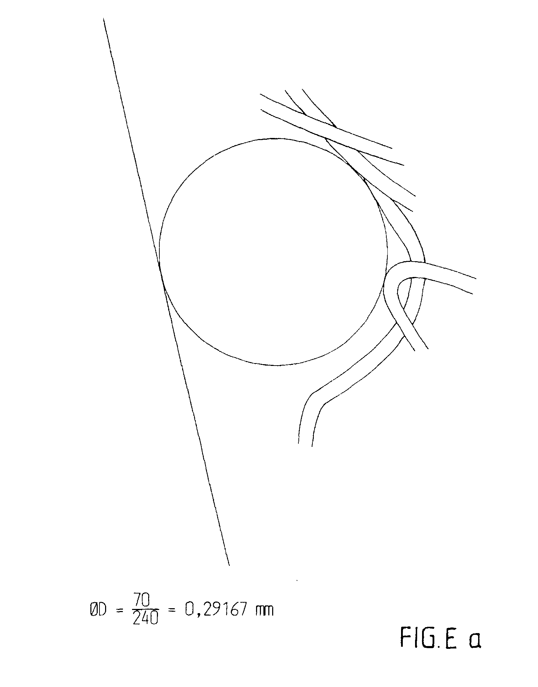

Figs. 11 and 12 are reproductions of photographs corresponding to those in Figs. 5 and 6 and show a single layer elastic film consisting of SBS. In this case, the photographs are also taken with the elastic film at an available elongation or stretch of 50% and 0% respectively (the film stretched to 100% and 150% respectively). No pores at all can be shown. Fig. 13 shows the same film enlarged 1000 times.

Still no pores can be observed. The pore radius is thus so small as to be unmeasurable.

The invention also relates to the use as a liquid barrier in absorbent articles 1, 20, 28, such as a diaper, sanitary napkin, incontinence guard or like articles, preferably diaper or incontinence guard of a web-like element 14 comprised of essentially liquid-impermeable material, wherewith the liquid barrier includes an elongated, essentially inelastic and essentially liquid-impermeable material 18, and a band-like elastic device 15 which is fastened in a stretched state to the material 18 such as to pucker said material, and so that a longitudinally extending part 17, 24 of said elastic device 15 remains free. The elastic device 15 has two longitudinal edges 16, 17, 21, 22 and a longitudinally extending free part 17, 24, 25. The liquid barrier also includes the web-like, essentially inelastic material 18 which has two longitudinally extending edges 19, 23, of which one edge 23 is fastened to the elastic device 15 and the other edge 19 is intended to be fastened to the absorbent article 1, 20, 28. All embodiments of the elements shown in Figs. 1b-f can be used in this respect.

A nonwoven material, e.g. a multi-ply nonwoven material, is an example of the essentially inelastic material that can be used. Such a material may be an SMS material, i.e. spunbond-melt-blown-spunbond.

The material used with the elastic device may be elastic film or elastic band based on styrene block copolymers, such as SBS (styrene-butadiene-styrene), SIS (styrene-isoprene-styrene), SEBS (styrene-ethylene-butylene-styrene) or SEPS (styrene-ethylene-propylene-styrene).

The film may consist of several layers, for instance it may be a three-ply film in which the outer plies consist of polypropylene, with the polypropylene functioning to facilitate fastening of the film to the inelastic material or to the absorbent article. Polypropylene also feels more comfortable to the skin than a number of other elastic films, which feel like plastic against the skin, which one wishes to avoid.

When a multi-layer film is used, it is possible for one of the outer layers to crack, or crackle, in the manner of the PP film in the three-ply film shown in Fig. 4. It is the crackled film that gives rise to the largest pores between the film and the wearer of the article. In order to obtain the smallest possible pores with such film, it is important that the outer layer that will lie against the wearer in use is so thin as not to give rise to unnecessarily large pores. This outer film layer will preferably not be thicker than 20 µm. The pore size ought then to be smaller than 20 µm. The pore size will be still smaller when the outer layer of the film does not crack.

Present-day absorbent articles include a liquid barrier that has an available elongation or stretch of about 10-50% on the wearer of the article. An available elongation or stretch beneath 10% is uncomfortable and corresponds roughly to the extent to which a tie diaper or diaper square can be stretched. In this embodiment of the inventive absorbent article, the elastic device has an available elongation or stretch of 10-80%, or preferably 15-50%.

In accordance with the invention, the aforesaid element includes an elastic device which has an essentially smooth surface in both a relaxed and a tensioned state and will present no pores, or particularly small pores, in the contact surface between the liquid barrier and the wearer's skin. The liquid barrier forms a seal with small pores irrespective of the extent to which the barrier can be stretched. The pores have a radius of at most 0.1 mm, preferably of at most 0.05 mm, or most preferably of at most 0.02 mm.

An important advantage afforded by the inventive absorbent article is thus that it prevents leakage when a given quantity of liquid, such as urine, floats on top of the upper sheet. And since the pore radius will remain essentially unchanged as the barrier is tensioned or relaxed, the same size of diaper can be used by persons of mutually different sizes, without changing the pore radius formed in the contact surface between the liquid barrier and the wearer's skin.

It will be understood that the invention is not restricted to the described and illustrated exemplifying embodiments thereof and that a number of modifications are conceivable within the scope of the following Claims.

## Claims

1. A web-like element (14) intended for use as a liquid barrier in absorbent articles (1, 20, 28), such as diapers, incontinence guards or like articles, wherein the element (14) includes an elongated essentially inelastic and essentially liquid-impermeable material (10, 18), and an elongated elastic device (13, 15) attached in a stretched state to the material (10, 18) such as to pucker said material, **characterized in that** the elastic device is a band-like device (15) having two longitudinally extending edges (16, 17, 21, 22) and fastened to the material (18) such that a longitudinally extending part (17, 24) of the elastic device (15) remains free.

2. An element according to Claim 1, **characterized in that** the free part (17, 24) of the elastic device has an essentially smooth surface in both a relaxed and a tensioned state.

3. An element according to Claim 1 or 2, **characterized in that** the elastic device (15) is fastened to the material (18) such that one edge (17) of said elastic device forms the longitudinally extending free part (17).

4. An element according to Claim 1 or 2, **characterized in that** both edges (21, 22) of the elastic device (15) are fastened to the material (18), wherewith the elastic device (15) includes a fold or bend (24) in the region between the two edges (21, 22), and wherewith said fold or bend (24) forms the longitudinally extending free part (24) of the elastic device.

5. An element according to any one of the preceding Claims, **characterized in that** the elastic device is comprised of an elastic film based on, e.g., SBS, SIS, SEBS or SEPS.

6. An element according to any one of the preceding Claims, **characterized in that** the essentially inelastic material is comprised of a nonwoven material, for instance a multi-layer nonwoven material, such as SMS or SM, or a hydrophobic polypropylene nonwoven material.

7. An element according to any one of the preceding Claims, **characterized in that** the elastic device has a width of at least 0.5 cm, preferably at least 1.0 cm.

8. An absorbent article (1, 20, 28) such as a diaper, an incontinence guard or like article, that includes longitudinally extending (6, 7) and transversely extending (8, 9) extremities, a bottom liquid-impermeable sheet (31), an absorbent sheet (32), an upper liquid-permeable sheet (30), wherein the articles includes at least one longitudinally extending liquid barrier (2, 3, 4, 5) comprised of essentially liquid-impermeable material (10) and fastened along or adjacent to the longitudinal extremities (6, 7) of the article on each side of the centre of the top sheet, said liquid barrier including a free, stretchable edge (12 ,17, 24, 25) intended to face towards the wearer in use, or the article include an upper liquid-impermeable sheet which is intended to lie against the wearer and which includes elastic for shaping the article to the wearer's body, wherewith the upper liquid-impermeable sheet is fastened to the bottom liquid-impermeable sheet at its outer extremities (6, 7, 8, 9) and wherewith the upper sheet includes at least one aperture for register with the anus and urethra orifice of a wearer, and wherewith the upper liquid-impermeable sheet includes elastic liquid barriers comprised of essentially liquid-impermeable material fastened around said aperture or apertures, **characterized in that** the liquid barrier includes an elongated, band-like elastic device (15) that has two longitudinally extending edges (16, 17, 21, 22) and includes a longitudinally extending free part (17, 24, 25) that forms the stretchable edge intended to lie against the wearer in use.

9. An absorbent article according to Claim 8, **characterized in that** the liquid barrier includes an elongated, essentially inelastic material (18) that has two longitudinally extending edges (19, 23), of which one edge (19) is fastened to the absorbent article (1, 20, 28) and the other edge (23) is fastened to the elastic device (15) with said device in a stretched state.

10. An absorbent article according to any one of Claims 8-9, **characterized in that** the free part (17, 24, 25) of the elastic device presents an essentially smooth surface in both a relaxed and a stretched state.

11. An absorbent article according to Claim 10, **characterized in that** the radius of the largest circle that can be enclosed in any through-penetrating pore formed in the contact surface between the edge of the liquid barrier and the user is essentially unchanged at different available elongations when the article is worn.

12. An absorbent article according to Claim 11, **characterized in that** the pore radius in the contact surface between the liquid barrier and the wearer is smaller than 0.10 mm, preferably smaller than 0.05 mm, and most preferably smaller than 0.02 mm, at an available elongation or stretch that lies within the range of 15-50%, or preferably at an available elongation or stretch of 10-80%.

13. An absorbent article according to any one of Claims 9-12, **characterized in that** the elastic device (15) is fastened to the material (18) such that one edge (17) of said elastic device will form said longitudinally extending free part (17).

14. An absorbent article according to any one of Claims 9-11, **characterized in that** both edges (21, 22) of the elastic device (15) are fastened to the material (18), wherewith the elastic device (15) presents in the region between said two edges (21, 22) a fold or bend (24) that forms the longitudinally extending free part (24) of said elastic device.

15. An absorbent article according to any one of Claims 8-14, **characterized in that** the elastic device is comprised of an elastic film based on, e.g., SBS, SIS, SEBS or SEPS, preferably SBS.

16. An absorbent article according to any one of Claims 9-15, **characterized in that** the essentially inelastic material is comprised of a nonwoven material, e.g. a multi-layer nonwoven material, such as SMS or SM, or a hydrophobic polypropylene nonwoven.

17. An absorbent article according to any one of Claims 8-16, **characterized in that** said article includes transverse liquid barriers fastened in the proximity of each of the transverse extremities (8, 9) of said article.

18. An absorbent article according to any one of Claims 8-17, **characterized in that** the elastic device has a width of at least 0.5 cm, preferably at least 1.0 cm.

19. The use as a liquid barrier in absorbent articles (1, 20, 28), such as diapers, incontinence guards or like articles of a web-like element (14) that includes an elongated, essentially inelastic and essentially liquid-impermeable material (18), and a band-like elastic device (15) which is fastened in a stretched state to the material (18) such as to pucker said material, and so that a longitudinally extending part (17, 24) of said elastic device (15) remains free.

## Patentansprüche

1. Gewebeartiges Element (14), das für die Verwendung als Flüssigkeitsbarriere in Absorptionsartikeln (1, 20, 28), wie z.B. Windeln, Inkontinenzschutzeinrichtungen oder ähnlichen Artikeln vorgesehen ist, wobei das Element (14) ein längliches im Wesentlichen nicht elastisches und im Wesentlichen flüssigkeitsundurchlässiges Material (10, 18) und eine längliche elastische Vorrichtung (13, 15) aufweist, die in einem gedehnten. Zustand an das Material (10, 18) derart angebracht ist, dass das Material sich faltet,
**dadurch gekennzeichnet, dass**
die elastische Vorrichtung eine bandartige Vorrichtung (15) mit zwei sich in Längsrichtung erstreckenden Rändern (16, 17, 21, 22) ist und an das Material (18) derart befestigt ist, dass ein sich in Längsrichtung erstreckender Teil (17, 24) der elastischen Vorrichtung (15) frei bleibt.

2. Element nach Anspruch 1,
**dadurch gekennzeichnet, dass**
der freie Teil (17, 24) der elastischen Vorrichtung sowohl im entspannten als auch gespannten Zustand eine im Wesentlichen glatte Oberfläche aufweist.

3. Element nach Anspruch 1 oder 2,
**dadurch gekennzeichnet, dass**
die elastische Vorrichtung (15) an das Material (18) derart befestigt ist, dass ein Rand (17) der elastischen Vorrichtung den sich in Längsrichtung erstreckenden freien Teil (17) bildet.

4. Element nach Anspruch 1 oder 2,
**dadurch gekennzeichnet, dass**
beide Ränder (21, 22) der elastischen Vorrichtung (15) an das Material (18) befestigt sind, wodurch die elastische Vorrichtung (15) eine Falte oder Biegung (24) in dem Bereich zwischen den beiden Rändern (21, 22) aufweist, und wodurch die Falte oder Biegung (25) den sich in Längsrichtung erstreckenden freien Teil (24) der elastischen Vorrichtung bildet.

5. Element nach zumindest einem der vorangehenden Ansprüche,
**dadurch gekennzeichnet, dass**
die elastische Vorrichtung aus einer elastischen Folie auf der Basis von beispielsweise SBS, SIS, SEBS oder SEPS besteht.

6. Element nach zumindest einem der vorangehenden Ansprüche,
**dadurch gekennzeichnet, dass**
das im Wesentlichen nicht elastische Element aus einem Vliesmaterial, beispielsweise einem Mehrschicht-Vliesmaterial, wie z.B. SMS oder SM oder einem wasserabweisenden Polypropylen-Vliesmaterial besteht.

7. Element nach zumindest einem der vorangehenden Ansprüche,
**dadurch gekennzeichnet, dass**
die elastische Vorrichtung eine Breite von wenigstens 0,5 cm, vorzugsweise wenigstens 1,0 cm aufweist.

8. Absorptionsartikel (1, 20, 28), wie z.B. Windel, Inkontinenzschutz oder ähnlicher Artikel, der sich in Längsrichtung erstreckende (6, 7) und in Querrichtung erstreckende (8, 9) Extremitäten, eine untere flüssigkeitsundurchlässige Lage (31), eine Absorptionslage (32), eine obere flüssigkeitsdurchlässige Lage (30) aufweist, wobei der Artikel wenigstens eine sich in Längsrichtung erstreckende Flüssigkeitsbarriere (2, 3, 4, 5) aufweist, die im Wesentlichen aus flüssigkeitsundurchlässigem Material (10) besteht und entlang oder an die Längsextremitäten (6, 7) des Artikels anliegend an jeder Seite der Mitte der Oberlage befestigt ist, wobei die Flüssigkeitsbarriere einen freien, dehnbaren Rand (12, 17, 24, 25) aufweist, der dafür vorgesehen ist, während der Verwendung zu dem Benutzer gerichtet zu. sein, oder der Artikel eine obere flüssigkeitsundurchlässige Lage aufweist, die dafür vorgesehen ist, gegen den Benutzer zu liegen und elastische Einrichtungen zum Formen des Artikels an den Körper des Benutzers aufweist, wodurch die obere flüssigkeitsundurchlässige Lage an ihren äußeren Extremitäten (6, 7, 8, 9) an die untere flüssigkeitsundurchlässige Lage befestigt ist, und wodurch die obere Lage wenigstens eine Öffnung aufweist, die mit dem Anus und der Harnröhrenöffnung eines Benutzers ausgerichtet ist, und wodurch die obere flüssigkeitsundurchlässige Lage elastische Flüssigkeitsbarrieren aufweist, die aus im Wesentlichen flüssigkeitsundurchlässigem Material bestehen, das um die Öffnung(en) befestigt ist,
**dadurch gekennzeichnet, dass**
dass die Flüssigkeitsbarriere eine längliche, bandartige, elastische Vorrichtung (15) aufweist, die zwei sich in Längsrichtung erstreckende Ränder (16, 17, 21, 22) und einen sich in Längsrichtung erstreckenden freien Teil (17, 24, 25) aufweist, der den dehnbaren Rand bildet, der dafür vorgesehen ist, während der Verwendung gegen den Benutzer zu liegen.

9. Absorptionsartikel nach Anspruch 8,
**dadurch gekennzeichnet, dass**
die Flüssigkeitsbarriere ein längliches, im Wesentlichen nicht elastisches Material (18) aufweist, das zwei sich in Längsrichtung erstreckende Ränder (19, 23) aufweist, von denen ein Rand (19) an den Absorptionsartikel (1, 20, 28) befestigt ist, und der andere Rand (23) an die elastische Vorrichtung (15), mit der elastischen Vorrichtung (15) in einem gedehnten Zustand, befestigt ist.

10. Absorptionsartikel nach einem der Ansprüche 8 bis 9,
**dadurch gekennzeichnet, dass**
der freie Teil (17, 24, 25) der elastischen Vorrichtung sowohl im entspannten als auch im gespannten Zustand eine im Wesentlichen glatte Oberfläche aufweist.

11. Absorptionsartikel nach Anspruch 10,
**dadurch gekennzeichnet, dass**
der Radius des größten Kreises, der in eine durchgehende Pore eingeschlossen werden kann, die in der Kontaktfläche zwischen dem Rand der Flüssigkeitsbarriere und dem Benutzer ausgebildet ist, an unterschiedlichen verfügbaren Verlängerungen im Wesentlichen unverändert ist, wenn der Artikel getragen wird.

12. Absorptionsartikel nach Anspruch 11,
**dadurch gekennzeichnet, dass**
der Porenradius in der Kontaktfläche zwischen der Flüssigkeitsbarriere und dem Benutzer kleiner als 0,10 mm, vorzugsweise kleiner als 0,05 mm und insbesondere kleiner als 0,02 mm ist, und zwar an einer verfügbaren Verlängerung oder Dehnung, die innerhalb eines Bereichs von 15-50% oder vorzugsweise bei einer verfügbaren Verlängerung oder Dehnung von 10-80% liegt.

13. Absorptionsartikel nach einem der Ansprüche 9 bis 12,
**dadurch gekennzeichnet, dass**
die elastische Vorrichtung (15) an das Material (18) derart befestigt ist, dass ein Rand (17) der elastischen Vorrichtung den sich in Längsrichtung erstreckenden freien Teil (17) bildet.

14. Absorptionsartikel nach einem der Ansprüche 9 bis 11,
**dadurch gekennzeichnet, dass**
beide Ränder (21, 22) der elastischen Vorrichtung (15)
an das Material (18) befestigt sind, wodurch die elastische Vorrichtung (15) in dem Bereich zwischen den beiden Rändern (21, 22) eine Falte oder Biegung (24) aufweist, die den sich in Längsrichtung erstreckenden freien Teil (24) der elastischen Vorrichtung bildet.

15. Absorptionsartikel nach einem der Ansprüche 8 bis 14,
**dadurch gekennzeichnet, dass**
die elastische Vorrichtung aus einer elastischen Folie auf der Basis von beispielsweise SBS, SIS, SEBS oder SEPS, vorzugsweise SBS besteht.

16. Absorptionsartikel nach einem der Ansprüche 9 bis 15,
**dadurch gekennzeichnet, dass**
das im Wesentlichen nicht elastische Material aus einem Vliesmaterial, beispielsweise einem Mehrschicht-Vliesmaterial, wie z.B. SMS oder SM oder einem wasserabweisenden Polypropylen-Vliesmaterial besteht.

17. Absorptionsartikel nach einem der Ansprüche 8 bis 16,
**dadurch gekennzeichnet, dass**
der Artikel Quer-Flüssigkeitsbarrieren aufweist, die in der Umgebung einer jeden der Querextremitäten (8, 9) des Artikels befestigt sind.

18. Absorptionsartikel nach einem der Ansprüche 8 bis 17,
**dadurch gekennzeichnet, dass**
die elastische Vorrichtung eine Breite von wenigstens 0,5 cm, vorzugsweise wenigstens 1,0 cm aufweist.

19. verwendung als eine Flüssigkeitsbarriere in Absorptionsartikeln (1, 20, 28), wie z.B. Windeln, Inkontinenzschutzeinrichtungen oder ähnlichen Artikeln eines gewebeartigen Elements (14), das ein längliches, im Wesentlichen nicht elastisches und im Wesentlichen flüssigkeitsundurchlässiges Material (18) und eine bandartige elastische Vorrichtung (15) aufweist, die in einem gedehnten Zustand an das Material (18) derart befestigt ist, dass das Material gefaltet wird, und so dass der sich in Längsrichtung erstreckende Teil (17, 24) der elastischen Vorrichtung (15) frei bleibt.

## Revendications

1. Elément de type voile (14) destiné à être utilisé comme barrière à liquide dans des articles absorbants (1, 20, 28), tels que des couches-culottes, des protections contre l'incontinence ou articles similaires, l'élément (14) comportant un matériau de forme allongée (10, 18) essentiellement inélastique et essentiellement imperméable aux liquides, et un dispositif élastique de forme allongée (13, 15) fixé au matériau (10, 18) dans un état étiré de façon à froncer ledit matériau, **caractérisé en ce que** le dispositif élastique est un dispositif en bande (15) ayant deux bords s'étendant longitudinalement (16, 17, 21, 22) et fixés au matériau (18) de telle manière qu'une partie s'étendant longitudinalement (17, 24) du dispositif élastique (15) reste libre.

2. Elément selon la revendication 1, **caractérisé en ce que** la partie libre (17, 24) du dispositif élastique a une surface essentiellement lisse, aussi bien dans un état relâché que dans un état tendu.

3. Elément selon la revendication 1 ou 2, **caractérisé en ce que** le dispositif élastique (15) est fixé au matériau (18) de telle manière qu'un bord (17) dudit dispositif élastique forme la partie libre s'étendant longitudinalement (17).

4. Elément selon la revendication 1 ou 2, **caractérisé en ce que** les deux bords (21, 22) du dispositif élastique (15) sont fixés au matériau (18), avec quoi le dispositif élastique (15) comporte un pli ou courbure (24) dans la région située entre les deux bords (21, 22), et avec quoi ledit pli ou courbure (24) forme la partie libre s'étendant longitudinalement (24) du dispositif élastique.

5. Elément selon l'une quelconque des revendications précédentes, **caractérisé en ce que** le dispositif élastique est constitué d'un film élastique, fabriqué par exemple à base de SBS, SIS, SEBS ou SEPS.

6. Elément selon l'une quelconque des revendications précédentes, **caractérisé en ce que** le matériau essentiellement inélastique est constitué d'un matériau non tissé, par exemple un matériau non tissé multicouche, tel que du SMS ou du SM, ou un matériau non tissé en polypropylène hydrophobe.

7. Elément selon l'une quelconque des revendications précédentes, **caractérisé en ce que** le dispositif élastique a une largeur d'au moins 0,5 cm, de préférence au moins 1,0 cm.

8. Article absorbant (1, 20, 28) tel qu'une couche-culotte, une protection contre l'incontinence ou article similaire, qui comporte des extrémités s'étendant longitudinalement (6, 7) et s'étendant transversalement (8, 9), une feuille inférieure (31) imperméable aux liquides, une feuille absorbante (32), une feuille supérieure (30) perméable aux liquides, l'article comportant au moins une barrière à liquide (2, 3, 4, 5) s'étendant longitudinalement, constituée d'un matériau (10) essentiellement imperméable aux liquides et fixée le long de ou adjacente aux extrémités longitudinales (6, 7) de l'article de part et d'autre du centre de la feuille supérieure, ladite barrière à liquide comportant un bond libre étirable (12, 17, 24, 25) destiné, en utilisation, à être tourné vers l'utilisateur, ou l'article comporte une feuille supérieure imperméable aux liquides qui est destinée à être au contact de l'utilisateur et qui comporte un élastique pour que l'article épouse la forme du corps de l'utilisateur, avec quoi la feuille supérieure imperméable aux liquides est fixée à la feuille inférieure imperméable aux liquides au niveau de ses extrémités extérieures (6, 7, 8, 9) et avec quoi la feuille supérieure comporte au moins une ouverture prévue pour être en correspondance exacte avec l'orifice de l'anus et de l'urètre d'un utilisateur, et avec quoi la feuille supérieure imperméable aux liquides comporte des barrières à liquide élastiques constituées d'un matériau essentiellement imperméable aux liquides fixé autour de ladite ou lesdites ouverture(s), **caractérisé en ce que** la barrière à liquide comporte un dispositif en bande, élastique et de forme allongée (15) qui a deux bords s'étendant longitudinalement (16, 17, 21, 22) et comporte une partie libre s'étendant longitudinalement (17, 24, 25) qui forme le bord étirable destiné, en utilisation, à être au contact de l'utilisateur.

9. Article absorbant selon la revendication 8, **caractérisé en ce que** la barrière à liquide comporte un matériau de forme allongée (18) essentiellement inélastique qui a deux bords s'étendant longitudinalement (19, 23), dont un bord (19) est fixé à l'article absorbant (1, 20, 28) et l'autre bord (23) est fixé au dispositif élastique (15), avec ledit dispositif dans un état étiré.

10. Article absorbant selon l'une quelconque des revendications 8 à 9, **caractérisé en ce que** la partie libre (17, 24, 25) du dispositif élastique présente une surface essentiellement lisse, aussi bien dans un état relâché que dans un état étiré.

11. Article absorbant selon la revendication 10, **caractérisé en ce que** le rayon du plus grand cercle qui peut être inscrit dans tout pore traversant formé dans la surface de contact entre le bord de la barrière à liquide et l'utilisateur est essentiellement inchangé à différents allongements possibles lorsque l'article est porté.

12. Article absorbant selon la revendication 11, **caractérisé en ce que** le rayon de pore dans la surface de contact entre la barrière à liquide et l'utilisateur est inférieur à 0,10 mm, de préférence inférieur à 0,05 mm, et mieux encore inférieur à 0,02 mm, à un allongement ou étirement possible qui se trouve dans la plage allant de 15 à 50 %, ou de préférence à un allongement ou étirement possible de 10 à 80 %.

13. Article absorbant selon l'une quelconque des revendications 9 à 12, **caractérisé en ce que** le dispositif élastique (15) est fixé au matériau (18) de telle manière qu'un bord (17) dudit dispositif élastique forme ladite partie libre s'étendant longitudinalement (17).

14. Article absorbant selon l'une quelconque des revendications 9 à 11, **caractérisé en ce que** les deux bords (21, 22) du dispositif élastique (15) sont fixés au matériau (18), avec quoi le dispositif élas tique (15) présente dans la région située entre lesdits deux bords (21, 22) un pli ou courbure (24) qui forme la partie libre s'étendant longitudinalement (24) dudit dispositif élastique.

15. Article absorbant selon l'une quelconque des revendications 8 à 14, **caractérisé en ce que** le dispositif élastique esl constitué d'un film élastique, fabriqué par exemple à base de SBS, SIS, SEBS ou SEPS, de préférence du SBS.

16. Article absorbant selon l'une quelconque des revendications 9 à 15, **caractérisé en ce que** le matériau essentiellement inélastique est constitué d'un matériau non tissé, par exemple un matériau non tissé multicouche, tel que du SMS ou du SM, ou un non tissé en polypropylène hydrophobe.

17. Article absorbant selon l'une quelconque des revendications 8 à 16, **caractérisé en ce que** ledit article comporte des barrières à liquide transversales fixées à proximité de chacune des extrémités transversales (8, 9) dudit article.

18. Article absorbant selon l'une quelconque des revendications 8 à 17, **caractérisé en ce que** le dispositif élastique a une largeur d'au moins 0,5 cm, de préférence au moins 1,0 cm.

19. Utilisation en tant que barrière à liquide, dans des articles absorbants (1, 20, 28) lets que des couches-culottes, des protections contre l'incontinence ou articles similaires, d'un élément de type voile (14) qui comporte un matériau de forme allongée (18) essentiellement inélastique et essentiellement imperméable aux liquides, et un dispositif élastique en bande (15) qui fixé au matériau (18) dans un état étiré de façon à froncer ledit matériau, et de telle manière qu'une partie s'étendant longitudinalement (17, 24) dudit dispositif élastique (15) reste libre.
